# EUROPEAN PATENT APPLICATION

(11) **EP 1 847 603 A1**
(43) Date of publication of application: **24.10.2007**
(21) Application number: 06712843.9
(22) Date of filing: 01.02.2006
(51) Int. Cl.: C12N 15/09, C12M 1/00, C12Q 1/68, G01N 33/53, G01N 37/00

(54) **METHOD FOR DETECTING p53 DYSFUNCTION, METHOD FOR MOLECULAR DIAGNOSIS OF CANCER AND METHOD FOR EVALUATING COMPOUND EFFECTIVE IN TREATING CANCER**

(30) Priority: 01.02.2005 JP 2005024905
(71) Applicant: BANYU PHARMACEUTICAL CO., LTD., Tokyo 102-8667 (JP)
(72) Inventor: KOTANI, Hidehito, c/o Banyu Pharmaceutical Co. Ltd, Tsukuba-shi, Ibaraki 3002611 (JP); YAMANAKA, Kazunori, c/o Banyu Pharma. Co. Ltd., Tsukuba-shi, Ibaraki 3002611 (JP); MIZUARAI, Shinji, c/o Banyu Pharmaceutical Co. Ltd, Tsukuba-shi, Ibaraki 3002611 (JP)
(74) Representative: Hussain, Deeba
(86) International application number: PCT/JP2006/301700
(87) International publication number: WO 2006/082866

(57) **Abstract**

Detection of p53 dysfunction or molecular diagnosis of cancer is carried out by a method comprising the steps of measuring the expression level of at least one gene selected from the group consisting of CARS, MOCOS, TNFRSF9, LOC56901 and GEF-H1 or a gene which is functionally equivalent to the gene in a test tissue or a test cell; comparing the expression level of the gene with that of a corresponding gene in a normal tissue or a normal cell; and determining whether or not the expression level of the gene in the test tissue or the test cell is significantly higher than that of the corresponding gene in the normal tissue or the normal cell based on the comparison result. According to the method for molecular diagnosis of the present invention, regardless of the presence of p53 mutation or without directly detecting p53 mutation, it becomes possible to detect p53 dysfunction or to diagnose cancer caused by p53 dysfunction.

## Description

### TECHNICAL FIELD

The present invention relates to a method for detecting dysfunction of a cancer suppressor gene p53 or for diagnosis of cancer by measuring expression of a predetermined gene. It also relates to a method of evaluating a compound based on the expression level of a predetermined gene.

### BACKGROUND ART

With recent development of Human Genome Project, pharmacogenomics is rapidly progressing that comprises understanding the individual difference in drug sensitivity on the genetic level to thereby apply it to development of drugs. When it becomes possible to determine the individual difference in drug sensitivity on the genetic level, then it may become possible to administer a drug having a strong side effect only to patients for which its efficacy can be expected and to carry out suitable medication from the initial stage of therapy without carrying out any trial-and-error medication, or that is, so-called personalized medicine may become possible. As a result, any useless physical burden may not be given to patients, and in addition, it may contribute toward reducing the medical cost recently increasing high. Above all, many anticancer agents often have an extremely strong side effect, with which, therefore, personalized medicine is desired.

When molecular level analysis of a disease-related gene of individuals becomes possible with respect to a disease that is the same between them but differs in the mechanism of the cause thereof, then therapy and prevention of the disease may become possible in accordance with the mechanism thereof.

On the other hand, p53 that is known as a cancer suppressor gene was at first discovered as a molecule that forms a hybrid with a T-antigen of a tumor virus SV40 (J. Virol., Vol. 31, p. 463, 1979: Non-Patent Reference 1). Afterwards, it has become clarified that the p53 gene is a cancer suppressor gene that exists in the short arm (17p13) of the 17th chromosome, as deleted in many cancers, and that the gene is inactivated through deletion and mutation along with its allele (N. Engl. J. Med., Vol. 319, p. 525, 1988: Non-Patent Reference 2). In addition, it has been clarified that its gene product is a transcriptional regulator comprising 393 amino acids, and forming a tetramer, it functions as it is. Further, an extremely large number of genes such as p21 and 14-3-3 that participate in a cell cycle, and bax, PIG3 and GADD45 that participate in apoptosis, have been identified as target genes to p53; and it is considered that the disorder of p53 may result in the disorder of transcriptional regulation of the target genes.

In fact, it has been clarified that the mutation of the p53 gene amounts to 70 % in lung cancer, 45 % in gastric cancer, 30 % in breast cancer, 65 % in colon cancer, 61 % in bladder cancer and 70 % in pancreas cancer (Experimental Medicine, Vol. 19, p. 135, 2001: Non-Patent Reference 3); and it is noticeably high, as compared with that of the other cancer suppressor genes. Specifically, it may be said that the dysfunction owing to the mutation of the p53 gene has a significant influence on carcinogenesis.

Given that situation, many studies relating to the relationship between the mutation or dysfunction of a p53 gene and diseases are made actively. For example, Soussi et al. made a database of p53 mutation of more than 15000 cases (http://p53.curie,fr/:Nucleic Acids Res., Vol. 22, p. 3551, 1994; Non-Patent Reference 4); indicating the occurrence of frequent mutation of codons 175, 245, 248 and 273 positioned within the DNA-binding domain region of p53. In addition, it has been clarified that, in a certain type of hepatocellular carcinoma, Arg to Ser mutation is admitted in the codon 249 in the DNA-binding domain (Nature, Vol. 350, p. 427, 1991: Non-Patent Reference 5). Further, it is reported that, also in a skin cancer caused by UV rays, CC in the p53 gene is thymine-dimerized (PNAS, Vol. 88, p. 10124, 1991: Non-Patent Reference 6).

Non-Patent Reference 1:J. Virol., Vol. 31, p. 463, 1979
Non-Patent Reference 2:N. Engl. J. Med., Vol. 319, p. 525, 1988
Non-Patent Reference 3: Experimental Medicine, Vol. 19, p. 135, 2001
Non-Patent Reference 4:Nucleic Acids Res., Vol. 22, p. 3551, 1994
Non-Patent Reference 5:Nature, Vol. 350, p. 427, 1991
Non-Patent Reference 6:PNAS, Vol. 88, p. 10124, 1991
Non-Patent Reference 7: Mol. Carcinogenesis, Vol. 19, p. 243, 1997

### DISCLOSURE OF THE INVENTION

However, p53 mutation does not always cause dysfunction or diseases, and even though mutated, it may have no influence on the function of p53 in some cases. For example, Li-QunJia et al. have clarified in their experiments with yeast that, though lymphoma-derived A3/KAW cells have missense mutation of codons 213 and 234 of p53 therein, no disorder is admitted in the transcriptional activity of the cells (Mol/ Carcinogenesis, Vol. 19, p. 243, 1997: Non-Patent Reference 7). In such a case, there is a problem in that, even p53 mutation could be detected, the relationship with dysfunction and diseases could not be presumed.

The present invention has been made in consideration of the above-mentioned problems of the prior art; and its object is to provide a method for detecting dysfunction of p53 and for effecting diagnosis of cancer caused by the dysfunction, regardless of the presence of p53 mutation or without directly detecting p53 mutation.

The present inventors have assiduously studied for the purpose of attaining the above-mentioned object, and as a result, have found that the expression level of a specific gene group changes, as linked to p53 dysfunction, and have completed the invention.

Specifically, the method for detecting p53 dysfunction of the invention is characterized by comprising a step of measuring the expression level of at least one gene selected from a group consisting of CARS, MOCOS, TNFRSF9, LOC56901 and GEF-H1 or a gene which is functionally equivalent to the gene in a test tissue or a test cell, a step of comparing the expression level of the gene with that of the corresponding gene in a normal tissue or a normal cell, and a step of determining whether or not the expression level of the gene in the test tissue or the test cell is significantly higher than that of the corresponding gene in the normal tissue or the normal cell based on the comparison result.

In this, the gene is preferably CARS or GEF-H1. Using the gene makes it possible to detect p53 dysfunction with higher accuracy.

The method for molecular diagnosis of cancer (method for evaluating or determining cancer) of the invention is characterized by comprising a step of measuring the expression level of at least one gene selected from a group consisting of CARS, MOCOS, TNFRSF9, LOC56901 and GEF-H1 or a gene which is functionally equivalent to the gene in a test tissue or a test cell, a step of comparing the expression level of the gene with that of the corresponding gene in a normal tissue or a normal cell, and a step of determining whether or not the expression level of the gene in the test tissue or the test cell is significantly higher than that of the corresponding gene in the normal tissue or the normal cell based on the comparison result, thereby expecting p53 dysfunction therein.

In this, the gene is preferably CARS or GEF-H1. Using the gene enables molecular diagnosis of cancer with higher accuracy.

The method for determining drug sensitivity of the invention is characterized by comprising a step of measuring the expression level of at least one gene selected from a group consisting of CARS, MOCOS, TNFRSF9, LOC56901 and GEF-H1 or a gene which is functionally equivalent to the gene in a test tissue or a test cell, a step of comparing the expression level of the gene with that of the corresponding gene in a normal tissue or a normal cell, and a step of determining whether or not the expression level of the gene in the test tissue or the test cell is significantly higher than that of the corresponding gene in the normal tissue or the normal cell based on the comparison result, thereby expecting p53 dysfunction therein.

In this, the gene is preferably CARS or GEF-H1. Using the gene makes it possible to determine drug sensitivity with higher accuracy.

The invention further includes a method of anticancer agent administration (method of anticancer agent selection) that comprises selecting the anticancer agent to be administered based on the result obtained in the above drug sensitivity determination method.

The cancer diagnostic kit (detection kit, detection kit for p53 dysfunction) of the invention is characterized by comprising an expression-measuring unit for measuring the expression of at least one gene selected from a group consisting of CARS, MOCOS, TNFRSF9, LOC56901 and GEF-H1, and a detection unit for detecting the expression of the gene.

In this, the expression-measuring unit is preferably any one selected from a group consisting of a microarray and a primer for PCR.

The method for evaluating a compound effective for cancer treatment of the invention is characterized by comprising a step of administering a test compound to a test animal or a test cell or contacting the compound with the animal or cell, a step of measuring the expression level of at least one gene selected from a group consisting of CARS, MOCOS, TNFRSF9, LOC56901 and GEF-H1 or a gene which is functionally equivalent to the gene in the test animal or the test cell, a step of comparing the expression level to that before administration of the test compound or contact with it, and a step of confirming a significant change in the expression level of the gene through the administration of the test compound or the contact with it based on the comparison result.

In this, the gene is preferably GEF-H1. Using the gene enables compound evaluation with higher accuracy.

The method for evaluating a compound effective for cancer treatment of the invention is characterized by comprising a step of administering a test compound to a test animal or a test cell or contacting the compound with the animal or cell, a step of measuring the activity of at least one protein selected from a group consisting of CARS, MOCOS, TNFRSF9, LOC56901 and GEF-H1 or a protein which is functionally equivalent to the protein in the test animal or the test cell, a step of comparing the activity to that before administration of the test compound or contact with it, and a step of confirming whether or not the protein activity has significantly changed before and after the administration or the contact based on the comparison result.

In this, the protein is preferably GEF-H1. Using the gene enables compound evaluation with higher accuracy.

The invention makes it possible to detect p53 dysfunction and to attain diagnosis of cancer caused by the dysfunction, without directly detecting p53 mutation or without giving a burden to a test person. In addition, the invention makes it possible to evaluate a compound effective for treatment of p53-associated cancer.

### BRIEF DESCRIPTION OF THE DRAWINGS

[Fig. 1] Fig. 1 is a view to confirm the expression of various recombinant p53 mutants on the level of protein.
[Fig. 2] Fig. 2 is a view to confirm the expression of various recombinant p53 mutants on the level of mRNA.
[Fig. 3] Fig. 3 is a view to show the relationship between various recombinant p53 mutants and p21 expression.
[Fig. 4] Fig. 4 is a view to show the relationship between the expression of various recombinant p53 mutants and the expression level of GEF-H1.
[Fig. 5] Fig. 5 is a view to show the relationship between GEF-H1 to be activated by p53 mutant expression induction, and RhoA to be activated by GEF-H1 expression.
[Fig. 6] Fig. 6 is a view to show the expression level of GEF-H1 in various cancer cells. In the drawing, the squares indicate cancer cells to express a mutant p53; and the triangles indicate cancer cell to express wild p53.
[Fig. 7] Fig. 7 is a view to show the cell viability in 3 days of wild p53-expressing HCT116 cells with the expression of GEF-H1 therein inhibited. The squares indicate a control (Scramble); and the lozenges indicate the viability of the cells with GEF-H1siRNA introduced thereinto.
[Fig. 8] Fig. 8 is a view to show the cell viability in 3 days of wild p53-expressing A427 cells with the expression of GEF-H1 therein inhibited. The squares indicate a control (Scramble); and the lozenges indicate the viability of the cells with GEF-H1siRNA introduced thereinto.
[Fig. 9] Fig. 9 is a view to show the cell viability in 3 days of wild p53-expressing RT4 cells with the expression of GEF-H1 therein inhibited. The squares indicate a control (Scramble); and the lozenges indicate the viability of the cells with GEF-H1siRNA introduced thereinto.
[Fig. 10] Fig. 10 is a view to show the cell viability in 3 days of mutant p53-expressing UMCU3 cells with the expression of GEF-H1 therein inhibited. The squares indicate a control (Scramble); and the lozenges indicate the viability of the cells with GEF-H1siRNA introduced thereinto.
[Fig. 11] Fig. 11 is a view to show the cell viability in 3 days of mutant p53-expressing SK-OV3 cells with the expression of GEF-H1 therein inhibited. The squares indicate a control (Scramble); and the lozenges indicate the viability of the cells with GEF-H1siRNA introduced thereinto.
[Fig. 12] Fig. 12 is a view to show the cell viability in 3 days of mutant p53-expressing ES-2 cells with the expression of GEF-H1 therein inhibited. The squares indicate a control (Scramble); and the lozenges indicate the viability of the cells with GEF-H1siRNA introduced thereinto.

### BEST MODE FOR CARRYING OUT THE INVENTION

Preferred embodiments of the invention are described in detail hereinunder.

The terms in the invention are first described.
"Test tissue" in the invention is a tissue capable of being extracted from a living body to be tested for cancer detection; and its type is not specifically defined so far as it is a cancer tissue that must be investigated for p53 participation in it, or a tissue that is considered necessary for its cancer diagnosis. Examples of the tissue include, for example, tissues derived from neuroblastoma, retinoblastoma, brain tumor, head and neck cancer, pituitary adenoma, glioma, acoustic schwannoma, oral cancer, pharyngeal cancer, laryngeal cancer, thyroid cancer, thymoma, mesothelioma, breast cancer, lung cancer, gastric cancer, esophageal cancer, colon cancer, hepatoma, pancreas cancer, pancreatic endocrine tumor, biliary cancer, penile cancer, vulvar cancer, ureteropelvic cancer, renal cancer, testicular cancer, prostate cancer, bladder cancer, uterine cancer, trophoblastic disorders, vaginal cancer, ovarian cancer, oviduct cancer, ovarian germ cell cancer, skin cancer, myocosis fungoides, malignant melanoma, soft tissue sarcoma, bone neoplasm, malignant lymphoma, leukemia, myelodysplastic dyndrome, multiple myeloma, lymphedema.

"Test cell" in the invention is also a tissue capable of being extracted from a living body to be tested for cancer detection; and its type is not specifically defined so far as it is a cancer tissue-derived cell that must be investigated for p53 participation in it, or a cell derived from a tissue that is considered necessary for its cancer diagnosis. Examples of the cell include, for example, cells derived from neuroblastoma, retinoblastoma, brain tumor, head and neck cancer, pituitary adenoma, glioma, acoustic schwannoma, oral cancer, pharyngeal cancer, laryngeal cancer, thyroid cancer, thymoma, mesothelioma, breast cancer, lung cancer, gastric cancer, esophageal cancer, colon cancer, hepatoma, pancreas cancer, pancreatic endocrine tumor, biliary cancer, penile cancer, vulvar cancer, ureteropelvic cancer, renal cancer, testicular cancer, prostate cancer, bladder cancer, uterine cancer, trophoblastic disorders, vaginal cancer, ovarian cancer, oviduct cancer, ovarian germ cell cancer, skin cancer, myocosis fungoides, malignant melanoma, soft tissue sarcoma, bone neoplasm, malignant lymphoma, leukemia, myelodysplastic dyndrome, multiple myeloma, lymphedema.

Next, the genes to be tested in the invention, CARS gene, MOCOS gene, TNFRSF9 gene, LOC56901 gene and GEF-H1 gene are described.
The protein encoded by CARS gene (cysteinyl-tRNA synthetase: NM_001751: SEQ ID NO. 1) is an enzyme existing in a cytoplasm, and this catalyzes aminoacylation of tRNA. It has been clarified that the CARS gene exists in 11p15.5 of a chromosome known as a site where a cancer suppressor gene exists (Genomics, Vol. 15, p. 692, 1993).

MOCOS gene (molybdenum cofactor sulfurase: NM_017947: SEQ ID NO. 2) was identified as a responsible gene for xanthinuria (Biochem. Biophys. Res. Commun., Vol. 282, p. 1194, 2001). MOCOS is a molecule comprising 2667 bases and 888 amino acids in the coding region thereof.

The protein encoded by TNFRSF9 gene (tumor necrosis factor receptor superfamily, member 9: NM_001561: SEQ ID NO. 3) belongs to a tumor necrosis factor (TNF) receptor family, and it is known to inhibit the growth of T cells and to induce apoptosis (Eur. J. Immunol., Vol. 9, p. 2219, 1994).

The protein encoded by GEF-H1 gene (Rho guanine exchange nucleotide factor-H1: NM_004723: SEQ ID NO. 4) is known as an enzyme that binds to Rho known as a cancer gene, and converts inactive Rho-GDP into active Rho (J. Biol. Chem., Vol. 273, p. 34954, 1998).

The protein encoded by LOC56901 gene (NM_020142: SEQ ID NO. 5) is known as a homologue to an oxidoreductase NDUFA4 that transport an electron from NADH to an electron transport chain (Proc. Natl. Acad. Sci. USA, Vol. 99, p. 16899, 2002).

CARS, MOCOS, TNFRSF9, LOC56901 or GEF-H1 used in the invention may be a gene functionally equivalent to those genes.

"Gene which is functionally equivalent to at least one gene selected from a group consisting of CARS, MOCOS, TNFRSF9, LOC56901 and GEF-H1" as referred to herein means a gene which differs from that gene in point of the base sequence thereof but shows relatively high homology to it, and encodes a protein that is the same as the protein of that gene or has a similar activity thereto. The homology as referred to herein is not specifically defined so far as the two are functionally equivalent to each other. Preferably, the homology of the base sequence is from 70 to 100 %, more preferably from 80 to 100 %, even more preferably from 90 to 100 %. When the homology is lower than the above lowermost limit, then there may be a high possibility that the gene in question would not show the same or similar function as or to the corresponding gene. However, even though the homology is lower than the above lowermost limit, the two genes may have the same or similar function in case where the homology between the domain having a function specific to the corresponding gene and the base sequence corresponding to that domain is high. Those genes may be preferably used even though the homology of the base sequence thereof is outside the above range. Genes having a relatively high homology may also be genes derived through spontaneous or artificial substitution, deletion, addition and/or insertion of one or more bases in CARS, MOCOS, TNFRSF9, LOC56901 or GEF-H1.

Next, the method for detection of p53 dysfunction of the invention is described.
The method for detection of p53 dysfunction of the invention is characterized by comprising a step of measuring the expression level of at least one gene selected from a group consisting of CARS, MOCOS, TNFRSF9, LOC56901 and GEF-H1 in a test tissue, a step of comparing the expression level of the gene with that of the corresponding gene in a normal tissue, and a step of determining whether or not the expression level of the gene in the test tissue is significantly higher than that of the corresponding gene in the normal tissue based on the comparison result.

In the invention, first, the expression level of at least one gene selected from a group consisting of CARS, MOCOS, TNFRSF9, LOC56901 and GEF-H1 in a test tissue is measured. Not specifically defined, the method for measuring the expression level of the gene includes, for example, a method of RT-PCR using the genomic DNA extracted from the test tissue as a template; a method of using a microarray where the gene is plotted; and northern blotting. In this, "expression level" of gene means an absolute amount or a relative amount of a genetic transcription product; and for the relative amount, the expression level of the gene may be determined with relative comparison to the expression level in a normal tissue to be described hereinunder.

In this step, the gene of which the expression level is to be determined is not limited to only one. The expression of two or more genes may be measured and the p53 dysfunction may be comprehensively determined from the result of the measurement.

Next, the expression level of the gene measured according to the above-mentioned method is compared with the expression level of the corresponding gene in a normal tissue.

"Normal tissue or normal cell" as referred to herein is not specifically defined in point of its origin, so far as it is a tissue or a cell of a subject to be compared with the test tissue or the test cell; and it may be a healthy person-derived one, or a cancer patient-derived one. It may be a normal tissue or a normal cell existing around a cancer tissue.

In this step, the expression level of the CARS, MOCOS, TNFRSF9, LOC56901 or GEF-H1 gene expressed in the test tissue or cell is compared with the expression level of that gene (corresponding gene) expressed in the normal tissue or cell, for which, the absolute amount of the expression level may be compared with each other, or the relative value may be computed through comparison.

Next, as a result of the comparison, it is determined whether or not the expression level of the gene in the test tissue or the test cell is significantly higher than that of the gene in the normal tissue or the normal cell.

The method for determination of the significant difference is not specifically defined, for which employable is a statistical test method known to those skilled in the art.

As a result of comparison between the expression level of the gene in the test tissue or the test cell and that in the normal tissue or the normal cell, in case where the expression in the test tissue or the test cell is recognized higher with a significant difference therebetween, then it may be determined that p53 dysfunction is recognized in the test tissue or the test cell. The cause of the p53 dysfunction in this case may include base deletion in p53 gene (for example, nonsense mutation), substitution (for example, missense mutation, point mutation), insertion, flame shifting. Regarding the subject of the invention, however, the cause of the dysfunction is not specifically defined, and includes any and every dysfunction caused by any of these.

Examples of the p53 dysfunction include reduction or activation of the transcriptional activity of p53 protein, or no transcription thereof at all, further including a case where there is no abnormality in the activity but the gene to be encoded is mutated.

Next, the method for molecular diagnosis of cancer (method for evaluating or determining cancer) of the invention is described.
The method for molecular diagnosis of cancer of the invention is characterized by comprising a step of measuring the expression level of at least one gene selected from a group consisting of CARS, MOCOS, TNFRSF9, LOC56901 and GEF-H1 in a test tissue or a test cell, a step of comparing the expression level of the gene with that of the corresponding gene in a normal tissue or a normal cell, and a step of determining whether or not the expression level of the gene in the test tissue or the test cell is significantly higher than that of the corresponding gene in the normal tissue or the normal cell based on the comparison result, thereby expecting p53 dysfunction therein.

Regarding the protocol of the method for molecular diagnosis of cancer of the invention, the process up to detection of p53 dysfunction is the same as that in the above-mentioned method for detection of p53 dysfunction. According to that method, in case where the p53 dysfunction is detected, then it may be determined that the test tissue or the test cell is a tissue or a cell derived from a cancer caused by p53 disorder. Specifically, in the tissue from which the test tissue or the test cell is derived, the species of the cancer that is known to be caused by p53 disorder may be specifically identified. Concretely, for example, the relationship between Li-Fraumeni syndrome (Science, Vol. 250, p. 1233, 1990), hepatocellular carcinoma (Nature, Vol. 350, p. 377, 1991), osteogenic sarcoma (Proc. Natl. Acad. Sci. USA, Vol. 84, p. 7716, 1987), rhabdomyosarcoma (Proc. Natl. Acad. Sci. USA, Vol. 87, p. 5863, 1990), colon cancer (Science, Vol. 244, p. 217, 1989), lung cancer (Science, Vol. 246, p. 491, 1989), glioblastoma (Am. J. Hum. Genet., Vol. 47 (suppl.), A4, 1990), esophageal cancer (Proc. Natl. Acad. Sci. USA, Vol. 87, p. 9958, 1990), bladder cancer (Science, Vol. 252, p. 706, 1991), squamous cell cancer (Proc. Natl. Acad. Sci. US, Vol. 88, p. 10124, 1991), cervical cancer (Lancet, Vol. 339, p. 1070, 1992), lung cancer (Proc. Natl. Acad. Sci. USA, Vol. 89, p. 7262, 1992), leukemia lymphoma ( J. Clin. Invest., Vol. 90, p. 653, 1992), which are suggested to participate in p53, and the test tissue and the test cell can be determined for cancer diagnosis.

Next, the method for determining drug sensitivity of the invention is described.
The method for determining drug sensitivity of the invention is characterized by comprising a step of measuring the expression level of at least one gene selected from a group consisting of CARS, MOCOS, TNFRSF9, LOC56901 and GEF-H1 in a test tissue or a test cell, a step of comparing the expression level of the gene with that of the corresponding gene in a normal tissue or a normal cell, and a step of determining whether or not the expression level of the gene in the test tissue or the test cell is significantly higher than that of the corresponding gene in the normal tissue or the normal cell based on the comparison result, thereby expecting p53 dysfunction therein.

Regarding the protocol of the method for molecular diagnosis of cancer of the invention, the process up to detection of p53 dysfunction is the same as that in the above-mentioned method for detection of p53 dysfunction. According to that method, in case where the p53 dysfunction is detected, then it may be determined that the test tissue or the test cell is a tissue or a cell derived from a cancer caused by p53 disorder. In other words, regarding a drug known in point of the relationship between its efficacy and p53, the drug sensitivity may be anticipated as to how and to what degree its efficacy can be expected when the drug is administered or as to what type of side effect may be caused by the drug administration. As a result, administration of a drug that is expected to cause some side effect is evaded, and a drug that is expected to be effective may be selectively administered. A drug known in point of the relationship between its efficacy and p53 includes, for example, doxorubicin-type or anthracycline-type anticancer agents; and a predetermined relationship is suggested between a lung cancer caused by the existence of genetic mutation in the DNA-binding region of p53 and the responsibility of those anticancer agents to it (Nat. Med., Vol. 2, p. 811, 1996). However, the drug to which the method for determining drug sensitivity of the invention is applicable is not limited to only the above-mentioned anticancer agents. In the invention, the relationship between any and every anticancer agent and p53 is investigated or researched, and the drug sensitivity may be thereby determined.

Specifically, in case where the test tissue or the test cell has been clarified to contain a cancer cell that has relation to p53, according to the method of determining drug sensitivity of the invention, then it may be possible to determine whether the administration of the anticancer agent to a subject case is effective, and it may be therefore possible to determine whether or not the drug is to be administered to the subject case. According to this determination, a suitable drug may be selected and administered, thereby enabling drug administration for high therapeutical effect with reducing its side effect.

The invention also provides a cancer diagnostic kit that comprises an expression-measuring unit for measuring the expression of at least one gene selected from a group consisting of CARS, MOCOS, TNFRSF9, LOC56901 and GEF-H1, and a detection unit for detecting the expression of the gene.

Not specifically defined, the expression-measuring unit for measuring the expression of at least one gene selected from a group consisting of CARS, MOCOS, TNFRSF9, LOC56901 and GEF-H1 includes, for example, a primer for amplifying the gene, a microarray plotted with the gene, and a probe for northern blotting. Also not specifically defined, the detection unit for detecting the gene expression includes, for example, a fluorescence-labeled reagent or a radioisotope-labeled reagent for visualizing the probe in northern blotting.

Using the cancer diagnostic kit enables simple and rapid diagnosis of p53-associated cancer, not requiring a step of detecting p53 mutation through extraction of the genome DNA from a test tissue or a test cell followed by confirmation of the base sequence thereof.

Next described is the method of compound evaluation of the invention.
The first embodiment of the method of compound evaluation of the invention is characterized by comprising a step of administering a test compound to a test animal or a test cell or contacting the compound with the animal or cell, a step of measuring the expression level of at least one gene selected from a group consisting of CARS, MOCOS, TNFRSF9, LOC56901 and GEF-H1 in the test animal or the test cell, a step of comparing the expression level of the gene to that of the corresponding gene in a normal animal or a normal cell, and a step of confirming a significant difference between the expression level of the gene in the test animal or the test cell and the expression level of the gene in the normal animal or the normal cell based on the comparison result.

Concretely, a compound is evaluated according to the following protocol.
First, a test compound is administered to or contacted with a test animal or a test cell. In this, the test compound may be any candidate compound for a drug that is intended for treatment, prevention or diagnosis of cancer, and is not specifically defined in point of its structure and properties and also in point of its species.

The method for administering the test compound to a test animal is not also specifically defined; and any favorable administration method may be selected in every case. For example, it includes oral administration and parenteral administration (e.g., endermic administration, intramuscular injection, intravenous injection, subcutaneous injection). The method for contacting the test compound with a test cell is not also specifically defined. For example, a cell and the test compound may be mixed in a solution such as a culture solution or a buffer solution (e.g., phosphate buffer solution), in which the two may be contacted with each other.

Next, the expression level of at least one gene selected from a group consisting of CARS, MOCOS, TNFRSF9, LOC56901 and GEF-H1 or a gene which is functionally equivalent to the gene in the test animal or the test cell is measured. The method for measuring the expression level of the gene is not specifically defined. Using the sample before administration or contact as a control, the change in the gene expression level is detected for comparison, according to a genetic amplification method such as RT-PCR, or a microarray method, or a northern blotting method. For the measurement of the expression level, usable is an animal or a cell into which a fused gene of the expression regulation region of the gene and a reporter gene has been artificially introduced. In this case, the reporter gene includes, for example, a β-galactosidase gene, a luciferase gene, a green fluorescence protein gene.

"Gene which is functionally equivalent to at least one gene selected from a group consisting of CARS, MOCOS, TNFRSF9, LOC56901 and GEF-H1" as referred to herein means a gene which differs from that gene in point of its base sequence but which has a relatively high homology to that gene and encodes a protein having the same or similar activity to that of the protein encoded by that gene. The homology as referred to herein is not specifically defined so far as the two are functionally equivalent to each other. Preferably, the homology of the base sequence is from 70 to 100 %, more preferably from 80 to 100 %, even more preferably from 90 to 100 %. When the homology is lower than the above lowermost limit, then there may be a high possibility that the gene in question would not show the same or similar function as or to the corresponding gene. However, even though the homology is lower than the above lowermost limit, the two genes may have the same or similar function in case where the homology between the domain having a function specific to the corresponding gene and the base sequence corresponding to that domain is high. Those genes may be preferably used even though the homology of the base sequence thereof is outside the above range. Genes having a relatively high homology may also be genes derived through spontaneous or artificial substitution, deletion, addition and/or insertion of one or more bases in CARS, MOCOS, TNFRSF9, LOC56901 or GEF-H1.

Next, the expression level of the gene measured in the previous step is compared with that before the administration of the test compound or the contact with it. Specifically, this step is to confirm how and to what degree the expression level of the test gene has changed before and after the administration of the test compound or the contact with it. The result of the comparison is determined whether or not it has any significant difference in the gene expression level; and in case where the expression level of CARS, MOCOS, TNFRSF9, LOC56901 or GEF-H1 is significantly high after the administration of the test compound or the contact with it, then it is determined that the effect of the compound is low; but when it is low, then it is determined that the compound is effective.

Next, the second embodiment of the compound evaluation method of the invention is described.
The second embodiment of the compound evaluation method of the invention is characterized by comprising a step of administering a test compound to a test animal or a test cell or contacting the compound with the animal or cell, a step of measuring the activity of at least one protein selected from a group consisting of CARS, MOCOS, TNFRSF9, LOC56901 and GEF-H1 or a protein which is functionally equivalent to the protein in the test animal or the test cell, a step of comparing the activity to that before administration of the test compound or contact with it, and a step of confirming whether or not the protein activity has significantly changed before and after the administration or the contact based on the comparison result.

Concretely, a compound is evaluated according to the following protocol.
First, a test compound is administered to or contacted with a test animal or a test cell. In this, the test compound may be any candidate compound for a drug that is intended for treatment, prevention or diagnosis of cancer, and is not specifically defined in point of its structure and properties and also in point of its species.

The method for administering the test compound to a test animal is not also specifically defined; and any favorable administration may be selected in every case. For example, it includes oral administration and parenteral administration (e.g., endermic administration, intramuscular injection, intravenous injection, subcutaneous injection). The method for contacting the test compound with a test cell is not also specifically defined. For example, a cell and the test compound may be mixed in a solution such as a culture solution or a buffer solution (e.g., phosphate buffer solution), in which the two may be contacted with each other.

Next, the activity of at least one protein selected from a group consisting of CARS, MOCOS, TNFRSF9, LOC56901 and GEF-H1 or a protein which is functionally equivalent to the protein in the test animal or the test cell is measured. The method for measuring the protein activity is not specifically defined. Using the sample before administration or contact as a control, for example, the aminoacylation activity of tRNA may be measured for CARS.

"Protein which is functionally equivalent to at least one protein selected from a group consisting of CARS, MOCOS, TNFRSF9, LOC56901 and GEF-H1" as referred to herein means a protein which differs from that protein in point of its amino acid sequence but which has a relatively high homology to that protein and has the same or similar activity to that of that protein. The homology as referred to herein is not specifically defined so far as the two are functionally equivalent to each other. Preferably, the homology of the amino acid sequence is from 50 to 100 %, more preferably from 60 to 100 %, even more preferably from 70 to 100 %. When the homology is lower than the above lowermost limit, then there may be a high possibility that the protein in question would not show the same or similar activity as or to the corresponding protein. However, even though the homology is lower than the above lowermost limit, the two proteins may have the same or similar activity in case where the homology between the domain having a function specific to the corresponding protein and the amino acid sequence corresponding to that domain is high. Those proteins may be preferably used even though the homology of the amino acid sequence thereof is outside the above range. Proteins having a relatively high homology may also be proteins derived through spontaneous or artificial substitution, deletion, addition and/or insertion of one or more amino acids in CARS, MOCOS, TNFRSF9, LOC56901 or GEF-H1.

Next, the activity of the protein measured in the previous step is compared with that before the administration of the test compound or the contact with it. Specifically, this step is to confirm how and to what degree the activity of the test gene has changed before and after the administration of the test compound or the contact with it. The result of the comparison is determined whether or not it has any significant difference in the activity; and in case where the activity of CARS, MOCOS, TNFRSF9, LOC56901 or GEF-H1 is significantly high after the administration of the test compound or the contact with it, then it is determined that the effect of the compound is low; but when it is low, then it is determined that the compound is effective.

According to the compound evaluation method of the invention mentioned above, compounds useful for cancer treatment or prevention may be screened, and the effectiveness and the safety of the drugs may be evaluated.

### EXAMPLES

The invention is described more concretely with reference to the following Examples, to which, however, the invention should not be limited.

First a mutant p53 expression vector plasmid was constructed.
A wild p53 gene and p53 gene with each mutation of V157F, R175H and R248Q were cloned into a doxocycline induction-type vector pTRE-hyg. Next, the p53 expression vectors were introduced into a cancer cell, U20S thereby establishing an induction-type p53 mutant expression cell line.

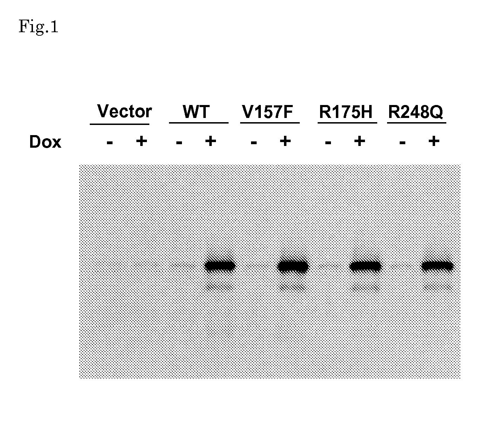

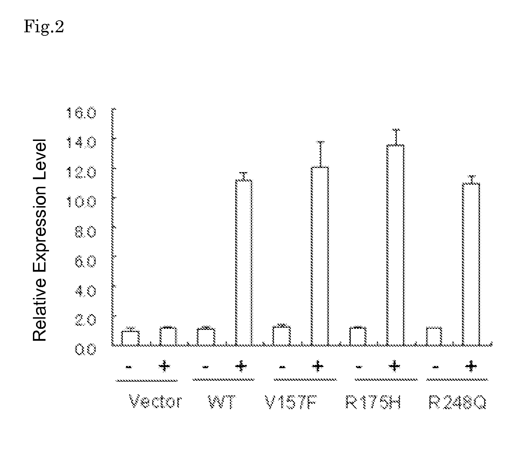

Next, expression of p53 in the wild p53 gene and each mutant p53 gene-having cell line was confirmed. The protein-level expression was measured according to a western blotting method (Fig. 1); and the mRNA-level expression was measured according to a real-time RT-PCR method (Fig. 2). The sequences of the primers and the probe used in RT-PCR are shown below.
Forward Primer: 5'-TCCCCGGACGATATTGAACA-3'(SEQ ID NO. 6)
Reverse Primer: 5'-GCATTCTGGGAGCTTCATCTG-3'(SEQ ID NO. 7)
TaqMan Probe: 5'-TGGTTCACTGAAGACCCA-3'(SEQ ID NO. 8)

As a result, after the induction, the expression induction of the wild and mutant p53 was 10 times or more.

Next, it was confirmed whether the endogenous p53 function was lost in these sell lines owing to the mutant p53 introduced thereinto.

First, the transcriptional activation potency of p53 was examined by measuring the p21 gene expression induction. The sequences of the primers and the probes used in the real-time RT-PCR method are shown below.
Forward Primer: 5'-TGGAGACTCTCAGGGTCGAAA-3' (SEQ ID NO. 9)
Reverse Primer: 5'-GGCGTTTGGAGTGGTAGAAATC-3' (SEQ ID NO. 10)
TaqMan Probe: 5'-CGGCGGCAGACCAGCATGAC-3' (SEQ ID NO. 11)

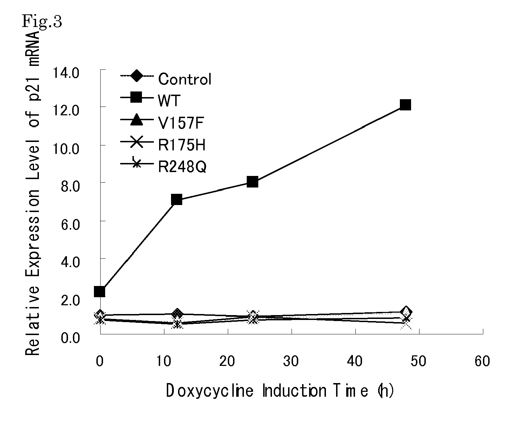

Doxocycline was added to the prepared cell line, and the wild and mutant p53 were induced therein. As a result, 10 hours after the induction, transcriptional activation of p21 by 10 times or more was seen in the wild p53 expression cell; but in the three mutant cells, no transcriptional activation of p21 was seen (Fig. 3). This confirms that the mutant p53 lost its transcriptional activation potency.

Next, for searching for a gene of which the expression is induced by mutant p53, cyclopedical gene expression of wild-type and mutant p53 expression cell lines and a control, U2OS cell line was measured using microarrays. 0, 12 or 15 hours after the mutant p53 expression induction, mRNA was extracted from each cell, converted into cDNA through reverse transcription, then hybridized with an oligonucleotide microarray (by Affymetrix) and analyzed it. A gene group of which the gene expression level increased in all the three mutant p53 expression cell lines was extracted as follows. Concretely, the samples were compared before mutant p53 induction and 15 hours after the induction, and a gene group having shown an expression level increase by at least 1.5 times in at least one mutant cell and having shown a significant expression level increase in all mutants was extracted. A condition of no gene expression level change owing to the wild p53 expression induction was given to the samples. Under the condition, the following 12 genes were extracted. Gene Symbols: C9orf48, CCNB1IP1, MRPL46, ZNF24, CARS, MOCOS, TNFRSF9, CARS, LOC56901, GEF-H1, SFPQ, CCNL1

The presence or absence of the expression promotion in the gene group may be useful for determining whether the p53 state is wild or mutant in a cancer cell. Further, a gene group having shown an gene expression level increase by at least 1.5 times owing to all the mutant p53 for the gene extraction standard value was extracted, and it included two, GEF-H1 (Rho guanine nucleotide exchange factor H1) and CARS (cysteinyl-tRNA synthetase).

Next, GEF-H1 was specifically noted, and GEF-H1 induction by mutant p53 was confirmed according to a real-time RT-PCR method. The sequences of the primers and the probe used are shown below.
Forward Primer: 5'-TGAGATGTACGAGGTGCACACA-3' (SEQ ID NO. 12)
Reverse Primer: 5'-CGCACGCTCTGCTGAATG-3' (SEQ ID NO. 13)
TaqMan Probe: 5'-CCGGGATGACCGGAGCACCTG-3' (SEQ ID NO. 14)

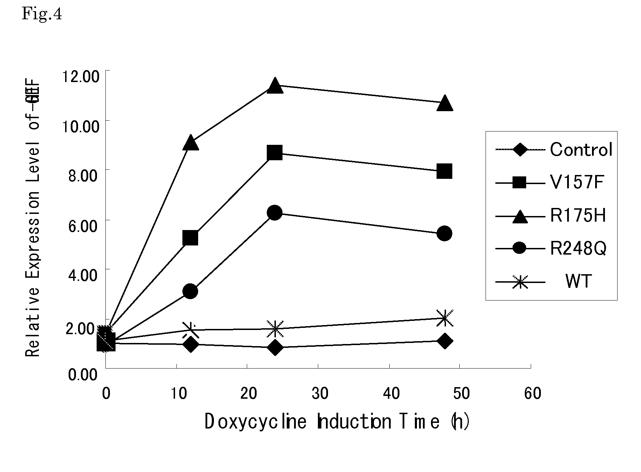

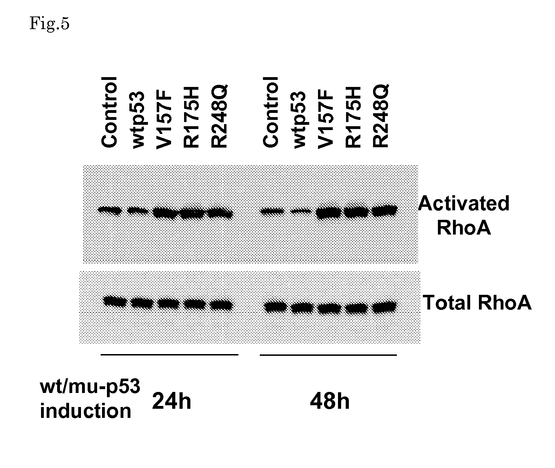

The time-dependent change in the GEF-H1 expression after p53 expression induction by doxycycline was determined. After 24 to 48 hours, the expression level was increased by at least 5 times in all the mutant p53 cell strains (Fig. 4). Further, since it is known that GEF-H1 activates a cancer gene RhoA, the RhoA activation condition after the mutant p53 expression induction was determined. A cell extract was prepared from each cell line in 24 and 48 hours after the mutant and wild p53 expression induction treatment, and the overall RhoA expression level and the active RhoA expression level were measured, using a kit (Rho activation assay kit, Cytoskeleton, Inc.) as follows. Since GST-Rhotekin-RBD (Rho-binding domain) binds to an active Rho, the sample was subjected to pull-down assay using GST-Rhotekin-RBD. The pulldown-assayed active GTPase was detected according to a western blotting method using a RhoA-specific antibody. As a result, it was clarified that, in the cell line in which mutant p53 was expressed and GEF-H1 was induced, the RhoA activity increased by at least 5 times (Fig. 5).

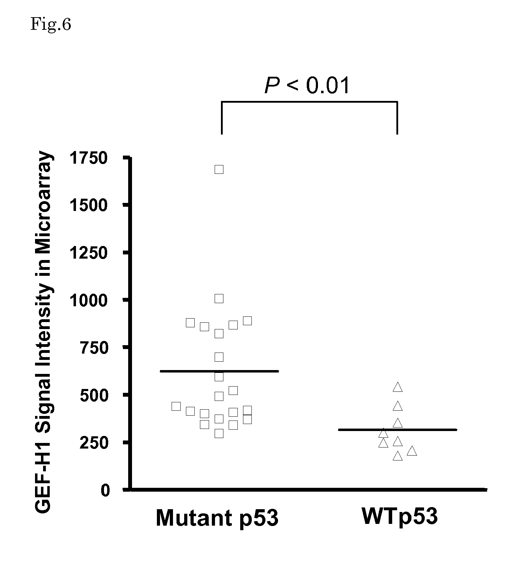

Next, the relationship between the p53 mutation and the GEF-H1 gene expression level in various cancer cells (29 types) was investigated. As in Fig. 6, it was clarified that the GEF-H1 gene expression level significantly increased in the mutant cell line group (p < 0.01).

Next, for investigating the carcinogenic promotion by the expression of GEF-H1 owing to the mutant p53 therein, the GEF-H1 expression was inhibited by siRNA in the wild and mutant p53 cell strains, and the cell growth potency of the cell lines was investigated. Concretely, siRNA (by Dharmacon) of GEF-H1 was transfected into a cancer cell, using oligofectamine; and after 3 days, the cell viability was determined according to an MTT method.

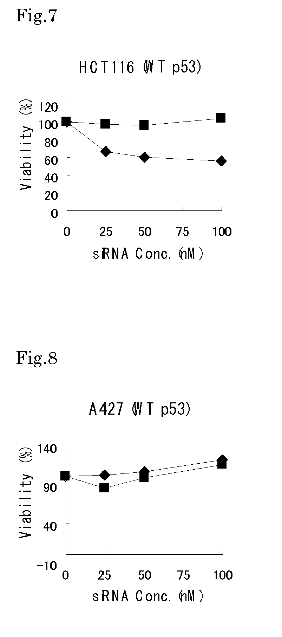

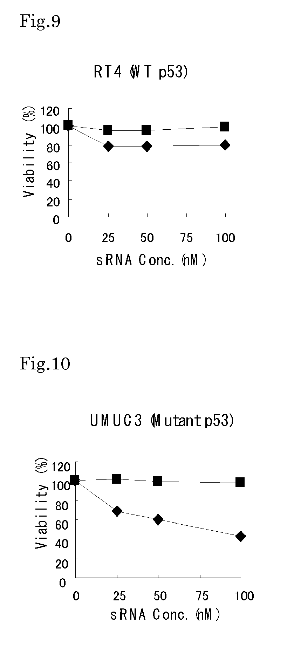

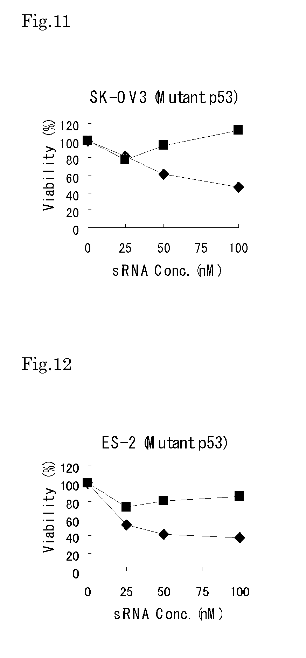

As in Figs. 7 to 9 (wild p53-expressing cancer cell lines) and Figs. 10 to 12 (mutant p53-expressing cancer cell lines), it was clarified that the cell growth potency significantly lowered owing to the GEF-H1 expression inhibition in the mutant p53-expressing cell group, as compared with that in the wild p53-expressing cell group.

### INDUSTRIAL APPLICABILITY

According to the invention, it is possible to detect p53 dysfunction and to diagnose cancer caused by the dysfunction, not directly detecting the mutation of p53 and not giving any load to a person to be tested. Further, according to the invention, it is possible to evaluate a compound effective for p53-associated cancer treatment.

## Claims

1. A method for detecting p53 dysfunction comprising:
a step of measuring the expression level (E₁) of at least one gene selected from a group consisting of CARS, MOCOS, TNFRSF9, LOC56901 and GEF-H1 or a gene which is functionally equivalent to the gene in a test tissue or a test cell,
a step of comparing the expression level (E₁) with the expression level (E₀) of the corresponding gene in a normal tissue or a normal cell, and
a step of determining whether or not the expression level (E₁) is significantly higher than the expression level (E₀) based on the comparison result.

2. The method for detecting p53 dysfunction as claimed in claim 1, wherein the gene is CARS or GEF-H1.

3. A method for molecular diagnosis of cancer comprising:
a step of measuring the expression level (E₁) of at least one gene selected from a group consisting of CARS, MOCOS, TNFRSF9, LOC56901 and GEF-H1 or a gene which is functionally equivalent to the gene in a test tissue or a test cell,
a step of comparing the expression level (E₁) with the expression level (E₀) of the corresponding gene in a normal tissue or a normal cell, and
a step of determining whether or not the expression level (E₁) is significantly higher than the expression level (E₀) based on the comparison result, thereby expecting p53 dysfunction therein.

4. The method for molecular diagnosis of cancer as claimed in claim 3, wherein the gene is CARS or GEF-H1.

5. A method for determining drug sensitivity comprising:
a step of measuring the expression level (E₁) of at least one gene selected from a group consisting of CARS, MOCOS, TNFRSF9, LOC56901 and GEF-H1 or a gene which is functionally equivalent to the gene in a test tissue or a test cell,
a step of comparing the expression level (E₁) with the expression level (E₀) of the corresponding gene in a normal tissue or a normal cell, and
a step of determining whether or not the expression level (E₁) is significantly higher than the expression level (E₀) based on the comparison result, thereby expecting p53 dysfunction therein.

6. The method for determining drug sensitivity as claimed in claim 5, wherein the gene is CARS or GEF-H1.

7. A method for selecting an anticancer agent, comprising selecting an anticancer agent to be administered based on the result obtained according to the drug sensitivity determination method of claim 5 or 6.

8. A cancer diagnostic kit comprising:
an expression-measuring unit for measuring the expression of at least one gene selected from a group consisting of CARS, MOCOS, TNFRSF9, LOC56901 and GEF-H1, and
a detection unit for detecting the expression of the gene.

9. The cancer diagnostic kit as claimed in claim 8, wherein the expression-measuring unit is any one selected from a group consisting of a microarray and a primer for PCR.

10. A method for evaluating a compound effective for cancer treatment comprising:
a step of administering a test compound to a test animal or a test cell or contacting the compound with the animal or cell,
a step of measuring the expression level of at least one gene selected from a group consisting of CARS, MOCOS, TNFRSF9, LOC56901 and GEF-H1 or a gene which is functionally equivalent to the gene in the test animal or the test cell,
a step of comparing the expression level to that before administration of the test compound or contact with it, and
a step of confirming a significant change in the expression level of the gene through the administration of the test compound or the contact with it based on the comparison result.

11. The evaluation method as claimed in claim 10, wherein the gene is GEF-H1.

12. A method for evaluating a compound effective for cancer treatment comprising:
a step of administering a test compound to a test animal or a test cell or contacting the compound with the animal or cell,
a step of measuring the activity of at least one protein selected from a group consisting of CARS, MOCOS, TNFRSF9, LOC56901 and GEF-H1 or a protein which is functionally equivalent to the protein in the test animal or the test cell,
a step of comparing the activity to that before administration of the test compound or contact with it, and
a step of confirming whether or not the protein activity has significantly changed before and after the administration or the contact based on the comparison result.

13. The evaluation method as claimed in claim 12, wherein the protein is GEF-H1.
